(19) **Europäisches Patentamt / European Patent Office / Office européen des brevets**

(11) **EP 4 686 485 A1**

(12) **EUROPEAN PATENT APPLICATION**

(43) Date of publication:
04.02.2026 Bulletin 2026/06

(21) Application number: 24315367.3

(22) Date of filing: 30.07.2024

(51) International Patent Classification (IPC):
*A61L 15/58* (2006.01)

(52) Cooperative Patent Classification (CPC):
(C-Sets available)
A61L 15/585 (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Advanced Silicone Coating
69330 Pusignan (FR)

(72) Inventors:
• Lecoeuvre, Jean-François
  69330 PUSIGNAN (FR)
• Guillermin, Mélanie
  69330 PUSIGNAN (FR)
• Jouvet, Jérome
  69330 PUSIGNAN (FR)
• Mathis, Laetitia
  69330 PUSIGNAN (FR)

(74) Representative: Ter Meer Steinmeister & Partner
Patentanwälte mbB
Nymphenburger Straße 4
80335 München (DE)

(54) **METHOD FOR THE PREPARATION OF A LAMINATE COMPRISING A SUBSTRATE AND A CROSSLINKED ADHESIVE MEDICAL SILICONE**

(57) The invention relates to a new method for preparing a laminate showing advantageous properties when being adhered to a surface such as skin and/or when being removed from a release liner. The laminate can for example be used as a wound contact layer for the preparation of articles such as wound dressings which are used for promoting/supporting the process of the healing of a wound.

EP 4 686 485 A1

(52) Cooperative Patent Classification (CPC): (Cont.)

C-Sets
**A61L 15/585, C08L 83/04**

**Description**

The invention relates to a new method for preparing a laminate showing advantageous properties when being adhered to a surface such as skin and/or when being removed from a release liner. The laminate can for example be used as a wound contact layer for the preparation of articles such as wound dressings which are used for promoting/supporting the process of the healing of a wound.

A wound can be regarded as the separation of the contiguity of tissues of the skin, wherein this can be combined with loss of substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step. A suitable laminate or wound dressing may help to provide a favourable environment for said healing process.

Such laminates have to be applied on a surface such as a wound or the skin surrounding said wound. For this purpose, it is essential that the laminate dressing adheres well to said surface to prevent an undesired slipping or even removal of the wound dressing. For storage as well as preparation purposes a laminate, before being applied to the wound, is often covered with a release liner which can be removed in the case of application. It turns out that it is important that the release force to remove such a release liner should neither be too high since otherwise its removal might damage the laminate nor too low since otherwise the release liner might at least partially peel off the laminate.

Within the field of laminates, polysiloxanes (herein referred to also as silicones), in particular crosslinked silicones, are known to have several advantages. For example, they exhibit adhesive and atraumatic properties and provide a pleasant skin feeling when applied to a wound.

There are several methods for preparing crosslinked silicones.

For example, prepolymers having each a functional group suitable to react with another can be subjected to a crosslinking or curing by radicals. A well-known radical starter is for example a peroxide. However, such a curing often produces odour- and/or flavour-impairing byproducts which makes it nearly impossible to be used for applications in the medical sector.

Further, the curing of the prepolymers can be conducted by catalysts. Such reactions are prone to small amounts of catalyst poisons, for example amine- and sulfur-containing compounds, in the ambient air, which can reduce or even inhibit the curing.

Curing or crosslinking of a silicone is not only accelerated by the presence of a catalyst but also by enhanced temperatures for example in the range of 120° to 200°C.

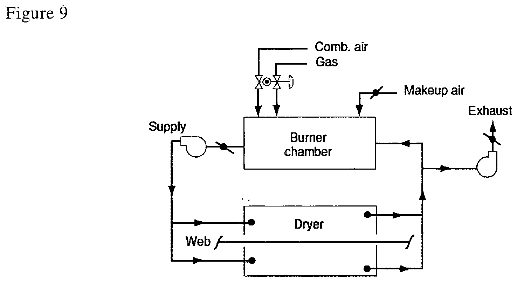

Therefore, a further method for preparing crosslinked silicone is the curing in a common convection oven in which a stream of hot air is blown at the surface of the material to be cured. The hot air can for example be produced by thermal exchange via steam or thermal oil or by direct heating by electricity or flame (natural gas burner). A typical system, a flotation dryer airflow system, is shown in Figure 9 below.

In this system, makeup air (fresh air) is heated by flame generated by a mix of combustion air and gas. The hot fresh air is blown onto the web coated with the mixture to be cured, wherein air temperature and velocity are main process factors. The quality/purity of the hot blown air blown onto the web with the mixture to be cured highly depends on the quality of combustion air and gas as well as the appropriate mixture thereof and further on the makeup air which is taken inside the plant where the reaction is carried out. A high purity of these different components can often not be achieved and thus there is a certain probability that the hot air contains inhibitors or poison for the catalysed reaction such as a Pt-catalysed hydrosilylation reaction. In addition, the described method seems to be improvable in view that the heat is transferred just to the top surface of the material to be cured and it is known that the thermal conductivity of silicone is rather low. Thus, there would be a temperature gradient in the material to be cured and the crosslinking might not be homogenous in the resulting silicone. Further, hot laminar air flow may be aggressive with the top surface of the material to be cured or the cured silicone which can create partial depolymerisation. Such depolymerisation can lead to poor cohesion in the top surface of the cured silicone. The poor cohesion and the too strong adhesiveness may be the reasons for 1) adhesive residues left on surfaces such as the skin when for example a wound dressing is removed and 2) converting issues when for example a release liner applied to the silicone is removed to manufacture dressings such as silicone residues on the liner, product deformation due to high liner release force, liner release force increasing over (storage) time, and problems while production, such as line speed reduction. In summary, the well-known curing process in a convection oven seems to be further improvable for example with regard to the process features and the properties of the resulting silicone.

Hence, it was an object of the present invention to overcome the above drawbacks.

In particular, it was an object of the present invention to provide a method for preparing a laminate comprising a crosslinked, adhesive medical silicone with a homogenous crosslinking throughout the whole material. In addition, a laminate comprising a crosslinked, adhesive medical silicone with homogenous viscoelastic properties should be achieved.

A further object of the present invention is to provide a laminate having good adhesive properties when applied to the skin and an appropriate, but not too high, and stable release force from the liner.

In addition, chemical inhibitions/contaminations and in the presence of undesired side reactions should be prevented or at least advantageously reduced.

Further, the use of high amounts of energy, $CO_2$ emissions, volatile organic compounds and heat emission into the workspace should be prevented or at least advantageously reduced as well.

### Summary of the invention

The present invention is suitable to solve one or more of the above-listed objectives by a specific method for preparing a laminate and by said laminate obtained by the present method.

Thus, the subject of the present invention is a method for preparing a laminate comprising a substrate and a crosslinked, adhesive medical silicone comprising the steps of:

(i) providing siloxane prepolymers and a catalyst,

(ii) blending the compounds from step (i),

(iii) applying the mixture resulting from step (ii) to a substrate, and

(iv) subjecting the mixture resulting from step (ii) to infrared (IR) radiation.

A further aspect of the present invention is a laminate obtainable by the present method.

Another aspect of the present invention is a medical product comprising the present laminate.

Another aspect is the use of an infrared radiation source for crosslinking prepolymers, preferably alkenyl-containing poly(organo)siloxane(s) and Si-H containing poly(organo)siloxane(s) to obtain an adhesive crosslinked polymer.

Finally, the present invention is directed to an apparatus comprising an infrared radiation and configured to carry out the present method.

### Figures

Figure 1 shows the tan delta, which is an indicator for the crosslinking degree of silicone obtained by curing in an oven or by different intensities of IR radiation.

Figure 2 shows the peel strength of crosslinked, adhesive silicones obtained by curing in an oven or by different intensities of IR radiation.

Figure 3 shows the release force of crosslinked, adhesive silicones obtained by curing in an oven or by different intensities of IR radiation.

Figure 4 shows the release force at high peel speed of crosslinked, adhesive silicones obtained by curing in an oven or by different intensities of IR radiation.

Figure 5 shows the release force of crosslinked, adhesive perforated silicones obtained by curing in an oven or by different intensities of IR radiation.

Figure 6 shows the release force of crosslinked, adhesive perforated and non-perforated silicones obtained by curing in an oven or by different intensities of IR radiation.

Figure 7 shows the peel curve of a silicone obtained by curing by IR radiation.

Figure 8 shows the peel curve of a silicone obtained by curing in an oven.

Figure 9 shows a flotation dryer airflow system as known in the art

**Detailed description**

The definitions are relevant in connection with the embodiments of the present invention.

The meaning of the term "comprising" is to be interpreted as encompassing all the specifically mentioned features as well as optional, additional unspecified ones, whereas the term "consisting of" only includes those features as specified. Therefore, "comprising" includes as a limiting case the meaning of "consisting of".

In this application the term "about" may be understood as +/- 5% of the corresponding value.

The term "% by weight" ("% w/w") is understood to refer to the weight amount of the component relative to the total weight of the adhesive silicone if not specified otherwise.

The meaning of the term "alkyl" is to be interpreted to encompass linear, branched and cyclic alkyl residues. Thus, for example an "alkyl having 1 to 3 carbon atoms" encompasses methyl, ethyl, propyl, isopropyl and cyclopropyl.

The meaning of the term "alkylene" is to be interpreted to encompass an alkyl which has at least one (unsaturated) double bond.

The present application is directed to a specific method for preparing a laminate comprising a substrate and a crosslinked adhesive medical silicone.

A laminate can be considered a product comprising two or more component's which are preferably planar. Said preferably planar components are bonded, preferably laminarly bonded, to each other.

A substrate can be referred to a substantially inert material which may be in form of a film.

A silicone can be regarded as polymers which comprise an silicon-oxygen ($\cdots$-Si-O-Si-O-Si-O-$\cdots$) chain as backbone wherein two organic groups are attached to each silicon, e.g. ($-R_2$Si-O-Si$R_2$-), wherein residue R is an organic group. A simple example is poly(dimethylsiloxane).

In the present invention the crosslinked, adhesive silicone is a medical silicone. A medical silicone can be referred to as silicone which has been tested and approved by the approval office. A medical silicone can be worn in contact with the body for a long period of time without causing any negative side effects to the environment/surface of the body to which it may be applied. It is hypoallergenic, resistant against bacteria growth and does not contain any harmful or toxic substances.

In the present invention the adhesive medical silicone is crosslinked. Thus, the silicone chains are crosslinked, i.e. there are single bond(s) or linker(s) containing preferably not more than 15 atoms which link the silicone chains with each other.

In the present invention, the crosslinked medical silicone is adhesive. This means that the silicone has the property that it can adhere to a surface of a body or that on one or more of its surfaces a body of the same or different characteristics can be adhered. In a preferred embodiment, the adhesiveness can be determined by the corresponding peel strength which can be regarded a measure for the bond strength between two surfaces. In a preferred embodiment of the present invention, after 24 hours of its preparation, the crosslinked medical silicone has a peel strength of 0.5 to 5.0 N/25 mm, preferably 1.5 to 3.0 N/25 mm, more preferably 1.7 to 2.7 N/25 mm, even more preferably 1.9 to 2.4 N/25 mm, in particular about 2.1 N/25 mm measured by FINAT FTM as described in the experimental section.

In step (i) of the method according to the present invention, siloxane prepolymers and a catalyst are provided.

The term prepolymer refers to a monomer or a system of monomers that have been reacted to an intermediate state such as an oligomer, wherein said compound still has reactive groups such that further reactions, such as further polymerization or crosslinking, can be carried out. Siloxane prepolymers suitable to form a crosslinked silicone are well known in the art.

In a preferred embodiment of the invention, the siloxane prepolymers are alkenyl-containing poly(organo) siloxane(s) and SI-H-containing poly(organo)siloxane(s).

An alkenyl-containing poly(organo)siloxane can be an alkenyl-terminated poly(organo)siloxane, a non-alkenyl-terminated poly(organo)siloxane or a mixture thereof.

An alkenyl-terminated poly(organo)siloxane has

iA) at least one terminal siloxyl unit represented by the Formula $(R^1)_2(R^2)SiO_{1/2}$, wherein $R^1$, which can be the same or different, are linear or branched alkyl groups with 1 to 6 carbon atoms or phenyl optionally substituted with linear or branched alkyl groups with 1 to 3 carbon atoms and $R^2$ is an alkenyl group having 2 to 6 carbon atoms, and

iiA) siloxyl units represented by the Formula $(R^3)_2SiO_{2/2}$, wherein $R^3$, which can be the same or different, are linear or branched alkyl groups with 1 to 6 carbon atoms or phenyl optionally substituted with linear or branched alkyl groups with 1 to 3 carbon atoms.

In a preferred embodiment, in the at least one terminal siloxyl unit represented by the Formula $(R^1)_2(R^2)SiO_{1/2}$, $R^1$ is the same and a linear or branched alkyl group with 1 to 3 carbon atoms or phenyl, preferably a linear or branched alkyl group with 1 to 3 carbon atoms, more preferably methyl.

In a preferred embodiment, in the at least one terminal siloxyl unit represented by the Formula $(R^1)_2(R^2)\,SiO_{1/2}$, $R^1$ is the same and a linear or branched alkyl group with 1 to 3 carbon atoms or phenyl, more preferably methyl, and $R^2$ is vinyl.

In a preferred embodiment, the alkenyl-terminated poly(organo)siloxane comprises two terminal siloxyl units represented by the Formula $(R^1)_2(R^2)\,SiO_{1/2}$, preferably two terminal siloxyl units, wherein $R^2$ is vinyl. Such alkenyl-terminated poly(organo)siloxane can also be also referred to as vinyl-terminated poly(organo)siloxane.

It is preferred that the alkenyl-terminated poly(organo)siloxane is substantially linear.

Non-alkenyl-terminated poly(organo)siloxane(s) have

iA) two terminal siloxyl units represented by the Formula $(R^1)_3\,SiO_{1/2}$, wherein $R^1$ can be the same or different, are linear or branched alkyl groups with 1 to 6 carbon atoms or phenyl optionally substituted with linear or branched alkyl groups with 1 to 3 carbon atoms, and

iiA) at least one siloxyl unit represented by the Formula $(R^2)(R^3)\,SiO_{2/2}$, wherein at least one of $R^2$ and $R^3$ is an alkenyl group having 2 to 6 carbon atoms and wherein, if not being an alkenyl group having 2 to 6 carbon atoms, the other of $R^2$ and $R^3$ can be a linear or branched alkyl group with 1 to 6 carbon atoms or phenyl optionally substituted with linear or branched alkyl groups with 1 to 3 carbon atoms aryl units.

In a preferred embodiment, in the terminal siloxyl units represented by the Formula $(R^1)_3\,SiO_{1/2}$, $R^1$ is the same and a linear or branched alkyl group with 1 to 3 carbon atoms or phenyl, preferably a linear or branched alkyl group with 1 to 3 carbon atoms, more preferably methyl.

In a preferred embodiment, in the at least one siloxyl unit represented by the Formula $(R^2)(R^3)\,SiO_{2/2}$, $R^2$ is vinyl and $R^3$ is a linear or branched alkyl group with 1 to 3 carbon atoms or phenyl, more preferably methyl.

In a preferred embodiment, the non-alkenyl-terminated poly(organo)siloxane(s) comprise(s) two siloxyl units represented by the Formula $(R^2)(R^3)\,SiO_{2/2}$, preferably two siloxyl units wherein $R^2$ is vinyl.

It is preferred that the non-alkenyl-terminated poly(organo)siloxane is substantially linear.

In a preferred embodiment, the alkenyl-containing poly(organo)siloxane is a mixture of different alkenyl-containing poly(organo)siloxane(s), preferably a mixture of two to five, more preferably two, different alkenyl-containing poly(organo)siloxane(s).

It is preferred that the alkenyl content, preferably the vinyl content, in the alkenyl-containing poly(organo)siloxane is 0.01 to 1, preferably 0.015 to 0.8 mmol/g of in the alkenyl-containing poly(organo)siloxane.

It is preferred that the alkenyl content, preferably the vinyl content, in the alkenyl-containing poly(organo)siloxane is 0.03 to 0.35%, preferably 0.05 to 0.30%.

It is preferred that the viscosity at 25°C in the alkenyl-containing poly(organo)siloxane is from 5000 to 20000 mPa·s, preferably from 8000 to 15000 mPa·s, in particular about 11000 mPa·s.

It is preferred that the alkenyl-containing poly(organo)siloxane(s) is an alkenyl-terminated poly(organo)siloxane.

An Si-H-containing poly(organo)siloxane can be an Si-H-terminated poly(organo)siloxane, a non-Si-H-terminated poly(organo)siloxane or a mixture thereof.

A Si-H-terminated poly(organo)siloxane has

iB) at least one terminal siloxyl unit represented by the Formula $(H)_q(R^4)_r SiO_{1/2}$, wherein $R^4$, which can be the same or different, are linear or branched alkyl groups with 1 to 6 carbon atoms or phenyl optionally substituted with linear or branched alkyl groups with 1 to 3 carbon atoms, q is selected from 1, 2 or 3 and q+r is equal to 3, and

iiB) siloxyl units represented by the Formula $(R^5)_2\,SiO_{2/2}$, wherein $R^5$, which can be the same or different, are linear or branched alkyl groups with 1 to 6 carbon atoms or phenyl optionally substituted with linear or branched alkyl groups with 1 to 3 carbon atoms.

In a preferred embodiment, in the at least one terminal siloxyl unit represented by the formula $(H)_q(R^4)_r SiO_{1/2}$, $R^4$ is the same and a linear or branched alkyl group with 1 to 3 carbon atoms or phenyl, preferably a linear or branched alkyl group with 1 to 3 carbon atoms, more preferably methyl.

It is further preferred that q is 1 or 2.

In a preferred embodiment in the siloxyl units represented by the formula $(R^5)_2\,SiO_{2/2}$, wherein $R^5$ are the same linear or branched alkyl groups with 1 to 3 carbon atoms or phenyl, preferably linear or branched alkyl groups with 1 to 3 carbon atoms, more preferably methyl.

A Si-H-non-terminated poly(organo)siloxane has

iB) two terminal siloxyl unit represented by the Formula $(R^4)_3\,SiO_{1/2}$, wherein $R^4$ can be the same or different, are linear or branched alkyl groups with 1 to 6 carbon atoms or phenyl optionally substituted with linear or branched alkyl groups with 1 to 3 carbon atoms, and

iiB) at least one siloxyl unit represented by the Formula $(H)_s(R^5)_tSiO_{2/2}$, wherein $R^5$, which can be the same or different, are linear or branched alkyl groups with 1 to 6 carbon atoms or phenyl optionally substituted with linear or branched alkyl groups with 1 to 3 carbon atoms, s is 1 or 2 and s+t is equal to 2.

In a preferred embodiment, in the at least one siloxyl unit represented by the Formula $(H)_s(R^5)_tSiO_{2/2}$, $R^5$ is the same linear or branched alkyl group with 1 to 3 carbon atoms or phenyl, preferably a linear or branched alkyl group with 1 to 3 carbon atoms, more preferably methyl.

It is further preferred that s is 1.

In a preferred embodiment, the Si-H containing poly(organo)siloxane is a mixture of different Si-H containing poly(organo)siloxanes, preferably a mixture of two to five, more preferably two, different Si-H containing poly(organo)siloxanes.

It is preferred that the Si-group content in Si-H containing poly(organo)siloxane is 0.5 to 20, more preferred 0.8 to 18, in particular 1.1 to 16 mmol/g of the Si-H containing poly(organo)siloxane(s).

It is preferred that the Si-group content in Si-H containing poly(organo)siloxane is 3.5 to 6.5, preferably 4.0 to 60%.

It is preferred that the viscosity at 25°C, in the Si-H containing poly(organo)siloxane is from 5000 to 30000 mPa·s, preferably 7500 to 25000 mPa.s, more preferably 10000 to 20000mPa·, in particular about 15000mPa·s.

In a preferred embodiment of the invention, siloxane prepolymers, preferably the alkenyl-containing poly(organo)siloxane and the Si-H containing poly(organo)siloxane, are present in a ratio of between 1 : 6.5 and 1 : 0.2, preferably between 1 : 5.5 and 1 : 0.4, in particular between 1: 4.5 and 1 : 0.6.

Further, a catalyst is provided in step (i). Generally, any catalyst suitable to carry out a crosslinking reaction, preferably a hydrosilylation reaction, can be used. Preferred are platinum-based catalysts. Non-limiting examples include platinum black, chloroplatinic acid, chloroplatinic acid modified with alcohol, olefin, aldehyde, preferably platinum acetyl acetonate and platinum alcoholates. Platinum can preferably be present in the corresponding catalyst in a content of between 5 to 10000 ppm. Further, the catalyst can preferably be used in an amount of between 0.001 to 0.5 by weight based on the weight of the sum of the siloxane prepolymers, preferably alkenyl-containing poly(organo)siloxane(s) and Si-H-containing poly(organo)siloxane(s).

In a preferred embodiment, the catalyst and one of the siloxane prepolymers, preferably the alkenyl-containing poly(organo)siloxane, can be provided as a mixture.

Further, in step (i) poly(organo)siloxane(s) not effecting the reaction of the siloxane prepolymers may be provided. Non-limiting examples of such poly(organo)siloxane(s) include poly(dimethylsiloxane) and alpha, omega-trimethylsiloxyl-poly(dimethylsiloxane).

Further, inhibitors suitable to avoid an undesired start of the reaction, to control the reaction and amount of crosslinking and/or to terminate the reaction may be provided in step (i). Such substances are known in the art. Non-limiting examples are tetramethyl vinyl tetrasiloxane, pyridine, phosphines and organic phosphines, unsaturated amides, alkyl maleates, preferably with an alkyl of 1 to 6 carbon atoms, acetylenic alcohols such as 1-ethynylclyclohexan-1-ol and generally substances bearing at least one alkenyl group. This substance can preferably be present in an amount of between 90 to 1100 ppm based on the weight of the sum of the alkenyl-terminated poly(organo)siloxane(s) and the Si-H containing poly(organo)siloxane(s).

Furthermore, one or more further substances, such as substances having an antimicrobial effect can be provided in step (i). Such substances are known in the art. Non-limiting examples include biguanide and derivatives thereof such as chlorhexidine, polyethylene biguanide (PEB), polytetramethylene biguanide (PTMB) or polyethylene hexamethylene biguanide (PEHMB); polyguanidine such as polyhexamethylene guanidine (PHMG), N-octyl-1-[10-(4-octyliminopyridin-1-yl)decyl]pyridin-4-imine (octenidine), quaternary ammonium compounds such as benzalkonium chloride or cetylpyridinium chloride; triazine such as 1-(3-chloroallyl)-3,5,7-triaza-1-azonia-adamantan chloride, and taurolidine.

In step (ii) the compounds from step (i) are blended.

"Blending" is understood in the context of the present invention as meaning a process of combining substances with the aim of achieving a substantially homogeneous distribution of different substances by the action of mechanical forces. Blending for the purposes of the invention is performed in conventional mixing devices such as tubular blenders, roll mixers, shaking mixers, free-fall mixers, shear mixers or intensive mixers. A tubular blender is preferably used.

Blending according to step (ii) may be conducted for example for 0.5 minutes to 1 hour, preferably for 2 minutes to 50 minutes, in particular for 5 minutes to 30 minutes, especially for about 10 minutes.

The optional one or more further substances such as inhibitor, substances having an antimicrobial effect and the optional poly(organo)siloxane(s) not effecting the reaction of the siloxane prepolymers can be added before, while or after blending the component(s) from step (i).

The blending from step (ii) results in a mixture comprising the compounds as described above.

In step (iii), the mixture resulting from step (ii) is applied to a substrate.

As indicated above, a substrate is a substantially inert substance. Non-limiting examples for a material of a substrate are materials/polymers such as polyethylene-based materials, polypropylene-based materials, polyester-based materials, such as materials from polyethylene terephthalate (PET), polyamide-based materials, polyvinylchloride-based materials, polyacrylate-based materials, polymethacrylate-based materials, polyurethane-based materials, such as materials from polyester urethane or polyether urethane, silicone-based materials and mixtures thereof. Preferred are polyurethane-based materials, such as materials from polyester urethane or polyether urethane.

The substrate can have any form as long as the mixture resulting from step (ii) can be applied on one side of said substrate. Preferably the substrate is a film. In a preferred embodiment the substrate, preferably the film, can have a thickness of 20 $\mu$m to 100 $\mu$m, more preferably from 30 $\mu$m to 90 $\mu$m, even more preferably from 40 $\mu$m to 80 $\mu$m, in particular from 50 $\mu$m to 70 $\mu$m, especially about 60 $\mu$m. Preferably the substrate, preferably the film, has a thickness of about 20 $\mu$m, about 30 $\mu$m, about 40 $\mu$m, about 50 $\mu$m, about 60 $\mu$m, about 70 $\mu$m, about 80 $\mu$m, about 90 $\mu$m, or about 100 $\mu$m. The substrate can be regarded as a carrier for the mixture resulting from step (ii).

Applying the mixture resulting from step (ii) to the substrate can be done by any method known in the art. The same applies to the device for applying the mixture resulting from step (ii) to the substrate. In a preferred embodiment, the mixture resulting from step (ii) can be coated with a coater on a substrate.

The mixture resulting from step (ii) can be applied to any percentage on one surface of the substrate. In a preferred embodiment, the mixture resulting from step (ii) can be applied to at least 50%, preferably at least 60%, more preferably at least 75%, in particular at least 90% of one surface of the substrate. Especially preferred, the mixture resulting from step (ii) can be applied to one substantially complete surface of the substrate.

The mixture resulting from step (ii) can be applied to the substrate in an amount of 10 to 500 g/m$^2$, preferably 25 to 400 g/m$^2$, more preferably 50 to 250 g/m$^2$, in particular 100 to 200 g/m$^2$, especially in an amount about 150 g/m$^2$.

In a preferred embodiment, the mixture resulting from step (ii) when applied to the substrate can be substantially evenly spread. In a preferred embodiment, the mixture resulting from step (ii) can be applied to the substrate in form of a layer. Said layer can have a thickness of from 20 $\mu$m to 225 $\mu$m, more preferably from 30 $\mu$m to 215 $\mu$m, in particular from 40 $\mu$m to 200 $\mu$m and especially from 90 $\mu$m to 200 $\mu$m. Preferably the adhesive silicone layer has a thickness of about 45 $\mu$m, about 50 $\mu$m, about 60 $\mu$m, about 70 $\mu$m, about 80 $\mu$m, about 90 $\mu$m, about 100 $\mu$m, about 110 $\mu$m, about 120 $\mu$m, about 130 $\mu$m, about 140 $\mu$m, about 150 $\mu$m, about 160 $\mu$m, about 170 $\mu$m, about 180 $\mu$m or about 190 $\mu$m.

In step (iv) of the present method, the mixture resulting from step (ii) is subjected to infrared (IR) radiation. Subjecting to the mixture resulting from step (ii) to infrared radiation is preferably carried out such that the infrared radiation is provided directly to the mixture resulting from step (ii) and does not have to pass the substrate or any other layer beforehand. Infrared radiation is generally referred to as a radiation with wavelengths longer than those of visible light but shorter than microwaves, i.e. a radiation with wavelengths from about 750 nm to about 1000 $\mu$m. In a preferred embodiment, the infrared radiation used in step (iii) is short-wavelength infrared or mid-wavelength infrared radiation. Short-wavelength infrared radiation includes wavelengths from about 750 nm to about 3 $\mu$m and mid-wavelength infrared radiation includes wavelengths from about 3 $\mu$m to 8 $\mu$m. Preferably, mid-wavelength infrared radiation is used in step (iv).

In an embodiment, the radiant power or radiant flux of the infrared radiation is from 15 to 70 kW, preferably from 20 to 55 kW, more preferably from 25 to 50 kW, even more preferably from 30 to 45 kW, especially about 35 kW, when applied by an infrared source in a distance of 20 cm to the mixture resulting from step (ii).

In another embodiment the radiant flux per area is from 7.5 to 35 kW/m$^2$, preferably from 10 to 25 kW/m$^2$, more preferably from 12.5 to 25 kW/m$^2$, even more preferably from 15 to 22.5 kW/m$^2$, especially about 17.5 kW/m$^2$.

Step (iv) may be carried out at an ambient temperature from 0°C to 50°C, preferably from 10°C to 40°C, more preferably from 15 to 35°C, in particular at about room temperature. 23°C and room temperature can be used interchangeably. Ambient temperature can be referred to as the temperature at the site where the method is carried out, for example a laboratory. The ambient temperature is usually significantly lower than the temperature in the mixture resulting from step (ii) while subjected to infrared radiation. Due to the power applied by the infrared radiation the temperature of said mixture can be significantly increased compared to the ambient temperature. In other words, due to the precise subjecting of the mixture resulting from step (ii) to infrared radiation, the heating of a complete reaction site such as an oven can be prevented.

The time for subjecting the mixture resulting from step (ii) to infrared radiation, preferably being carried out at a temperature of from 0°C to 50°, may take for example 0.5 to 30 seconds, preferably 1 to 25 seconds, more preferably 2 to 20 seconds, in particular about 5 seconds.

Subjecting the mixture resulting from step (ii) to infrared radiation can be carried out batchwise. This means that infrared radiation is applied to a certain area of the mixture resulting from step (ii) and subsequently the corresponding area of the resulting laminate can be further processed.

The method according to the present invention can be carried out batchwise or as a continuous process.

Batchwise means that the mixture resulting of step (ii) is applied to a substrate of a certain size and subsequently subjected to infrared radiation (cf. steps (iii) and (iv)). Subsequently, the resulting laminate of a certain size can be stored or further processed for example by kiss-cutting and/or by being covered with a release lines.

Preferably, the method according to the present invention can be carried out in a continuous process. For this purpose, the substrate can be conveyed at a certain line speed by a conveying device. Suitable conveying devices are known in the art. While being conveyed, the mixture resulting from step (ii) can be applied on the substrate by an application device. Application devices are known in the art. Examples are coater, nozzles and jets. Subsequently, the applied mixture can be preferably evenly spread, for example by a scraper or a doctor blade. Then, the substrate on which the mixture resulting from step (ii) can be further conveyed to pass an infrared source where step (iv) can be carried out. The resulting laminate can be further processed as indicated above.

A further subject of the present invention is a laminate obtainable by the method according to the present invention. According to one embodiment, the laminate is in form of a gel. In line with the present application a gel refers to a disperse system containing at least two components. The solid component can be regarded as a three-dimensional network having pores which are filled with a liquid such as water or a gas.

In a preferred embodiment, the laminate according to the invention has perforation holes, wherein the perforation holes are evenly distributed over the whole area of the layer. A perforation hole can be considered as an opening going through the layer. Evenly distributed over the whole area of the layer means that every part of the area has the same amount (in number and area) of openings. Thus, there is no part of the area which has more and/or bigger openings than another part. Preferably, the distance between all evenly distributed openings is constant or substantially the same, thereby creating a pattern of evenly distributed openings. The distance may be for example 0.1 to 5 mm, more preferably 0.3 to 3 mm and most preferably 0.4 to 2 mm.

Perforation holes can be made by any device suitable for the perforation of a layer such as nails, needles, laser, or a punching device. Alternatively, perforation can be carried out by die cutting, kiss cutting and by ultrasonic cutting.

The opening of a perforation hole can be any form. In other words, the form of the opening of a perforation hole can for example be rectangular, square, circular, elliptic, triangular, pentagonal, hexagonal, octagonal or diamond-shaped. In a preferred embodiment the openings of the perforation holes are square, circular, or elliptic, preferably circular.

The openings of the perforation holes have a size of between 1.75 $mm^2$ and 12.5 $mm^2$, preferably between 2.0 $mm^2$ and 8.5 $mm^2$, in particular between 2.25 $mm^2$ and 5.0 $mm^2$.

In case that the perforation hole is circular, the opening size of a perforation hole between 1.75 $mm^2$ and 12.5 $mm^2$ substantially corresponds to a diameter of the opening hole of about 1.5 mm to about 3.9 mm. Correspondingly, again if circular, the opening size of a perforation hole between 2.25 $mm^2$ and 5.0 $mm^2$ substantially corresponds to a diameter of the opening hole of about 1.7 mm to about 2.5 mm. A a perforation with the above size holes enables that the wound can be kept moist to improve the healing process and that the exudate can be removed from the wound itself, wherein it is additionally ensured that the formation of new formed cell attachment through the layer to the optional overlying layers, such as a wound pad layer, is significantly reduced or even prevented.

In a preferred embodiment of the invention, the perforated surface is between 10% and 50% of the total surface of the laminate. In other words, the sum of the areas of the opening of the perforation holes is between 10% and 50%, preferably between 12% and 45%, more preferably between 15% and 30% of the total surface of the layer. These areas can also be referred to as open (or opened) areas of the layer due to the absence of any material in this area.

The sum of the areas of the opening of the perforated holes is preferably about 15%, about 16%, about 17%, about 18%, about 19%, about 20%, about 21%, about 22%, about 23%, about 24%, about 25%, about 26%, about 27%, about 28%, about 29% or about 30%, more preferably about 25% of the total surface of the laminate.

A further subject of the present invention is a medical product comprising the laminate according to the invention. Non-limiting examples of medical products are diapers, patches, bandages and wound dressings. In a preferred embodiment, the medical product is a wound dressing.

In a further embodiment, the medical product comprises the laminate of the present invention and a further layer comprising absorbent material.

An absorbent material can be referred to as a material capable of receiving and subsequently retaining a liquid such as water or exudate. Absorbent materials are known in the art. Non-limiting examples include compounds based on natural polymeric material such as starch, dextran, agarose, pectin, alginate, chitosan, hyaluronic acid, gellan, polypeptide and cellulose, wherein these natural polymeric materials can be further processed, for example by chemical derivation such as the formation of esters and ethers or pharmaceutically acceptable salts and compounds based on synthetic polymeric material such as polyvinyl alcohol, polyalkylene oxide, poly(meth)acrylate, poly(eth)acrylate, poly alkyl(meth) acrylate, poly alkyl(meth)acrylate, vinyl polymer, polycaprolactam and polycaprolactone, polyurethane, polyurea-based and polyurethane-polyurea-copolymers. In an embodiment the absorbent material is a superabsorbent material based on a copolymer of acrylic acid and acrylamide or a foam, preferably a foam based on polyurethane, polyurea-based and polyurethane-polyurea-copolymers. The layer comprising absorbent materials has desorption properties, i.e. that said layer may desorb liquids such as water.

In a preferred embodiment of the invention, the crosslinked silicone, preferably the crosslinked silicone layer, of the laminate is the wound contact layer. A wound contact layer can be regarded as the layer being directly in contact with the wound. It is further preferred that the further layer comprising an absorbent material is arranged in contact with the crosslinked silicone layer of the present laminate, for example via perforation holes.

Such an arrangement can for example have the advantage that exudate can penetrate the crosslinked, adhesive silicone layer and subsequently absorbed by the further layer comprising absorbing material. It is further preferred that the further layer comprising absorbent material such as a foam can preferably have a surface being smaller than the surface of the crosslinked, adhesive silicone layer. It is further preferred that the further layer of absorbent material is arranged on or adhered to the centre of the crosslinked, adhesive medical silicone layer such that the four rims of the crosslinked, adhesive medical silicone layer are not covered by the further layer comprising absorbent material and which may be substantially of the same area size, thereby forming a continuous margin around the centre. Wound dressings having such a continuous margin are defined as island dressings. Such an arrangement enables the adhesion of another layer such as a backing layer to the side opposite to the wound contact layer such that the top surface of the wound dressing is covered by the backing layer.

A further aspect of the invention is the use of an infrared radiation source for crosslinking siloxane prepolymers, preferably alkenyl-containing poly(organo)siloxane(s) and Si-H-containing poly(organo)siloxane(s) to obtain a crosslinked, adhesive medical polymer. As far as the infrared radiation source and the siloxane prepolymers are concerned, the same applies as described above. In a preferred embodiment of this aspect, the crosslinked, adhesive medical polymer is comprised by a medical product. As far as medical products are concerned, the same applies as described above.

Another aspect of the invention is an infrared radiation source configured to carry out the present method.

**Exemplary section**

**Equipment:**

Convection oven:

Type: air flotation oven with a gas burner as heat source

Zoning: 3 zones (3 m, 3 m and 5 m)

Process parameters: speed line, temperature and velocity at the nozzles

Infrared equipment from Sopara (F):

Type: Medium wave infrared source

Panel dimension: 1000 mm in machine direction x 1850 mm in cross direction

Power: tunable up to 100 kW; i.e 100% correspond to 100 kW

**Product assessment:**

Crosslinking degree

The crosslinking degree as determined by the following viscoelasticity analysis:
The crosslinked, adhesive silicone was removed from the substrate to form a ball of 0.80 $\pm$0.01 g. This was placed between the geometries before the viscoelastic analysis.

The viscoelasticity analyses to determine the crosslinking degree were carried out with a Kinexus (rheometer from NETZCH) with the following parameters:

| | |
|---|---|
| Plan / plan geometry: | diameter of 20mm |
| Gap: | 1500 $\mu$m |
| Temperature: | 23°C |
| Constraint mode; frequency sweep | 1 to 30 Hz |

The result was obtained as tan $d_{test}$ corresponding to G"/G' (viscous modulus /elastic modulus) for 10 Hz. tan $d_{test}$ was compared to tan $d_{control}$ which is a value coming from a sample coated and cured at laboratory condition of 10 min for 140°C. Subsequently the crosslinking degree is calculated as follows

$$\text{Crosslinking degree \% = tan } d_{control} \text{ / tan } d_{test} \cdot 100\%$$

As can be seen from the above, the lower tan $d_{test}$ the higher the crosslinking degree.

Peel strength determination

The peel strength was determined by a method based on FINAT FTM 1 using a test specimen of 200 mm x 25 mm and Oxford Bristol paper as test panel. The samples were stuck on the test panel at a length of 80 mm. To obtain intimate contact between the crosslinked adhesive silicone and the surface of the Oxford Bristol paper, it was rolled twice in each direction with a FINAT test roller at a speed of about 10 mm /s. The FINAT test roller has a roll with a diameter $85 \pm 2.5$ mm, a roll cover of rubber with a hardness scale Shore A of $80 \pm 5$, a width of $50 \pm 1$ mm and a weight of $2 \pm 0.05$ kg. After rolling twice the test was done immediately at 300 mm / min with a peel angle of 180°.

PE-liner release force determination

The PE-liner release force was determined by a method based on FINAT FTM 3 using a test specimen of 200 mm x 25 mm and a separation test from the PE-Liner at 300 mm / min with a T shape.

**1. Preparation of a laminate layer according to the description**

1.1 100 g vinyl-terminated poly(organo)siloxane having a viscosity of about 11000 mPa·s and a 0.005g platinum catalyst (platinum acetyl acetonate) are provided as Part A.
Further, 100 g Si-H containing poly(organo)siloxane having a viscosity of about 15000 mPa·s are provided as Part B.
1.2 Parts A and B were blended to obtain a silicone mixture. Further a 25μm PU film was provided, wherein on one surface of said PU film a 30 μm acrylate adhesive was applied which, in turn, was covered on its opposite surface with a 90 μm releasable paper ("release paper"). On the other surface of said PU film a silicone primer was applied. While being conveyed with a line speed of 12 m/min, the mixture containing parts A and B was applied in a continuous process via the blade of an industrial coating machine in an amount of 140 g/m$^2$ on the silicone primer of the before described PU film to pass the above-described infrared equipment from Sopara such that the silicone mixture was cured. Table 1 shows the process parameters, wherein the velocity is the air velocity from the air coming out the nozzles.

Table 1

| | Coat weight [g/m$^2$] | Line speed [m/min] | IR power [%] | Oven zone 1 [Temp.] [velocity] | Oven zone 2 [Temp.] [velocity] | Oven zone 3 [Temp.] [velocity] |
|---|---|---|---|---|---|---|
| control | | Coating & cured in lab with same raw materials | | | | |
| Test 1 | 140 | 12 | 30 | Off | Off | Off |
| Test 2 | | | Off | 145°C 30 m/s | 140°C 30 m/s | 145°C 30 m/s |
| Test 3 | | | 30 | 145°C 30 m/s | 140°C 30 m/s | 145°C 30 m/s |

Table 2 shows the results:

| | Control | Test 1 | Test 2 | Test 3 |
|---|---|---|---|---|
| Tan d at 0°C | 0.68 | 0.80 | 0.88 | 0.77 |
| Crosslinking at 0°C [%] | 100 | 85.0 | 77.3 | 88.3 |
| Tan d after curing at 140°C for 5 min | - | 0.69 | 0.72 | 0.73 |

(continued)

|  | Control | Test 1 | Test 2 | Test 3 |
|---|---|---|---|---|
| Crosslinking after curing at 140°C for 5 min [%] | - | 98.6 | 94.4 | 93.2 |

As can been seen, when subjected to infrared radiation the mixture is cured to about 85%, but a crosslinking degree of almost 100% can be achieved by post curing. Without being bound to any theory, this is might be due to the absence of an inhibition or chemical poising during the reaction.

When subjected to temperatures in an oven, crosslinking of about 77% is achieved, but the crosslinking degree can only be enhanced up to about 84% by post curing. Again, without being bound to any theory, this might be due to the presence of an inhibition or chemical poising during the reaction in the oven.

When subjected to infrared radiation and temperatures in an oven, crosslinking of about 88% is achieved, but the crosslinking degree can only be enhanced up to about 93% by post curing. Again, without being bound to any theory and similar to the above, this might be due to the quality of the hot air blown onto the mixture.

1.3 Parts A and B were blended to obtain a silicone mixture. Further a 25 μm PU film was provided, wherein on one surface of said PU film a 30 μm acrylate adhesive was applied which, in turn, was covered on its opposite surface with a 90 μm releasable paper ("release paper"). On the other surface of said PU film a silicone primer was applied. While being conveyed with a line speed of 12 m/min, the mixture containing parts A and B was applied in a continuous process via the blade of an industrial coating machine in an amount of 140 g/m$^2$ on the silicone primer of the before-described PU film to pass the above-described infrared equipment from Sopara such that the silicone mixture was cured. Table 3 shows the process parameters.

Table 3

| Samples | Line speed [m/min] | IR Power [%] | Temp. Zone 1 [°C] | Temp. Zone 2 [°C] | Temp. Zone 3 [°C] | Air velocity Zone 1 [m/s] | Air velocity Zone 2 [m/s] | Air velocity Zone 3 [m/s] |
|---|---|---|---|---|---|---|---|---|
| IR 1 | 12 | 25 | Off | | | | | |
| IR 2 | 12 | 35 | | | | | | |
| ZF' | 12 | Off | 140 | Off | Off | 30 | Off | Off |
| ZF" | 12 | | Off | 140 | Off | Off | 30 | Off |
| ZF" | 12 | | Off | Off | 140 | Off | Off | 140 |

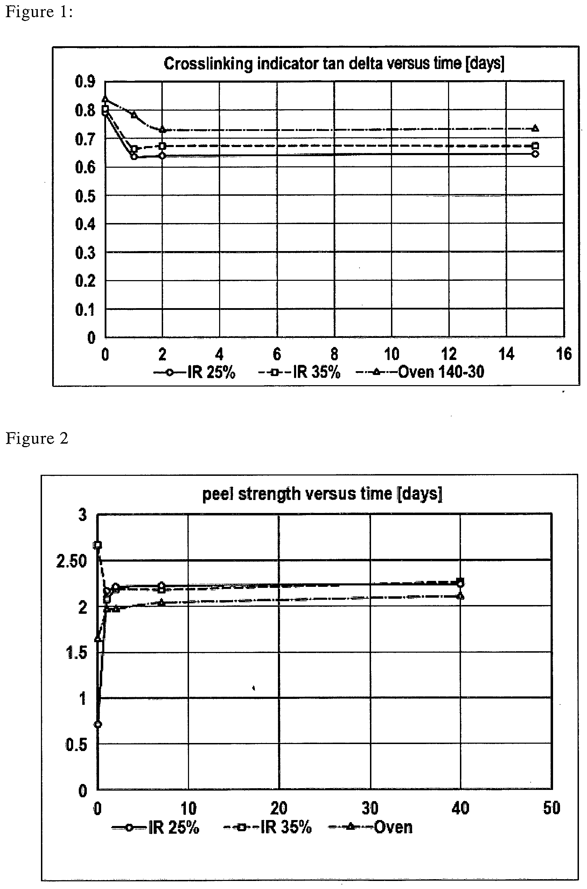

Subsequently, the tan delta (crosslinking indicator) of the test samples was determined and is shown in Figure 1. As can been seen from said Figure 1, the tan delta of sample ZF (Oven 140-30) is higher than the ones of IR 1 (IR 25%) and IR 2 (IR 35%). Thus, in view that the crosslinking is inversely proportional, the crosslinking degree of sample ZF using a conventional technique is lower than the ones cured/crosslinked with IR radiation

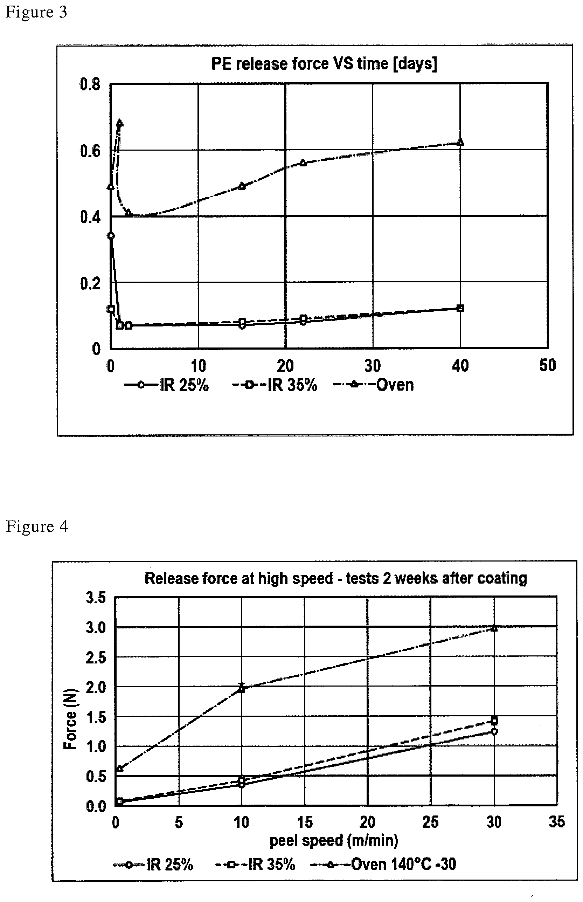

Further, a after the coated web exits from the oven, a PE-liner was applied in a continuous process, wherein the thickness of the PE-liner is 70 μm and subsequently, the peel strength and the PE-liner release force of the test samples were determined and shown in Figures 2 and 3.

Further, after a stabilisation period of about one day, the peel strength of samples IR 1 (IR 25%) and IR 2 (IR 35%) is higher than the one of sample ZF (Oven 140-30). A higher peel strength ensures a good adhesion of the adhesive layer of the substrate for example to the skin,

Furthermore, after a stabilisation period of about one and a half days, the PE-liner release force of samples IR 1 (IR 25%) and IR 2 (IR 35%) is lower than the one of sample ZF (Oven 140-30). This means that the PE-liner, which for example was applied for storage reasons, can be removed easily and thus ensuring that the substrate, in particular the crosslinked adhesive medical silicone thereof, is not damaged.

As an additional test, the high-speed release force was determined in accordance FINAT FTM 4 two weeks after a PET-release liner was applied. The parameters were as follows:

Test bench:          AR 2000
Test specimen:       300 mm x 25 mm

The result is the average release force of the curve and shown in Figure 4. As can be seen from said Figure, at high peel speed the release force of samples IR 1 (IR 25%) and IR 2 (IR 35 %) is about a third of the one of sample ZF (Oven 140-30). As discussed above, this enables an easy release of the PE-Liner from the substrate.

Perforation process impact and removal of perforated PE-liner ("release force")

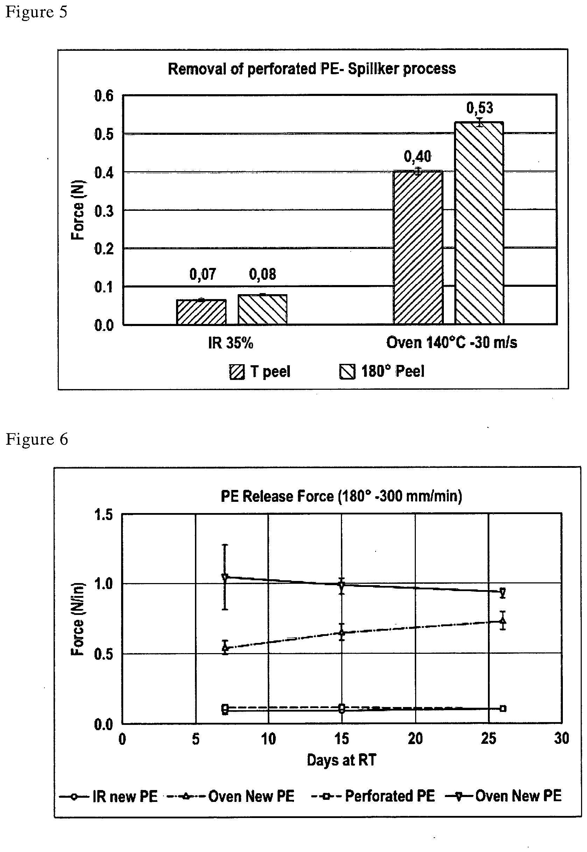

A. Two weeks after having been coated with a PE-liner, kiss-cutting perforation of the corresponding substrate with a small Spilker unit was carried out to obtain a perforated material (diameter of the whole of 2.4 mm and 15% open area of the complete surface). The release force measurement was carried out at 300 mm/min (T peel and 180° peel angle) and the result is shown in Figure 5. As can been seen from this Figure, the release force of IR 2 (IR 35%) is lower than the one of sample ZF (Oven 140-30). This enables an easier perforation process as the release force is a limiting factor of liner speed and a higher release force increases the presence of perforation defects.

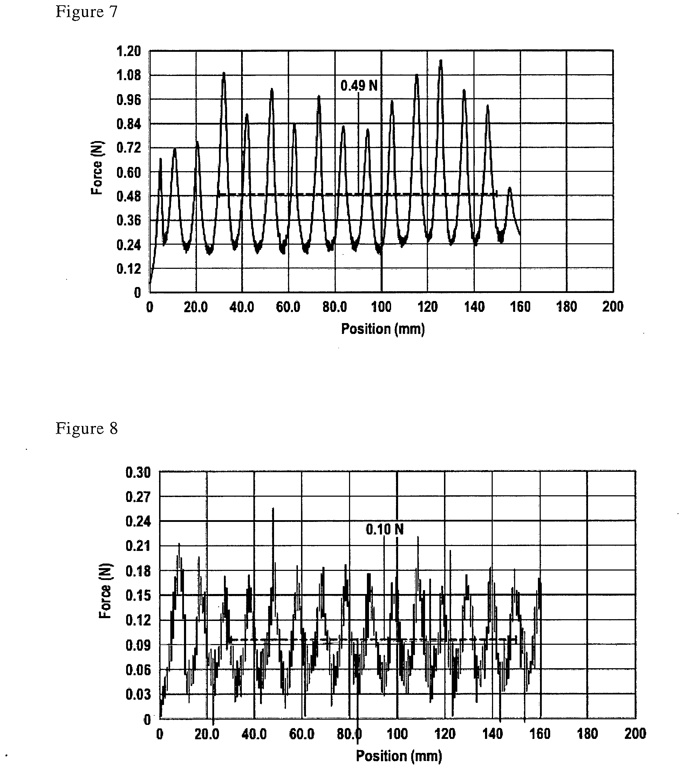

B. Two weeks after having been coated with a PE-liner, kiss-cutting perforation of the corresponding substrate with a small Spilker unit was carried out to obtain a perforated material (diameter of the whole of 2.4 mm and 15% open area of the complete surface) for each curing type. The types were samples with PE-liners replacing the perforated PE-liners and samples with perforated PE-liners. After one week at room temperature, the release force measurement was carried out at 300 mm/min (180° peel angle) and the results are shown in Figures 6 to 8. As can been seen from Figure 6, oven curing leads to high release forces and Figure 7 shows high peeks and thus gel elongation at the end of the perforation holes, in other words, high force variations during peeling (plain, hole, plain, hole etc.) In summary, oven curing results in a poor peel mode with silicone residue on the removed PE-liners. On the contrary, IR curing leads to significantly lower release forces (cf. Figure 7) and from Figure 8 it can be seen that there are low release force variations during peeling and no silicone residue on the removed PE-liner. Thus, the processability is significantly improved.

## Claims

1. Method for preparing a laminate comprising a substrate and a crosslinked adhesive medical silicone, wherein the method comprises the steps of:

   (i) providing siloxane prepolymer(s) and a catalyst,
   (ii) blending the compounds from step (i),
   (iii) applying the mixture from step (ii) to a substrate, and
   (iv) subjecting the mixture resulting from step (ii) to infrared (IR) radiation.

2. Method according to claim 1, wherein the crosslinked adhesive medical silicone has a peel strength of 0.5 to 5N/25mm measured by the method based on FINAT FTM as described in the description.

3. Method according to claim 1 or 2, wherein the siloxane prepolymers are alkenyl-containing poly(organo)siloxane(s) and Si-H containing poly(organo)siloxane(s).

4. Method according to any one of claims 1 to 3, wherein the catalyst is platinum-based catalyst.

5. Method according to any one of claims 1 to 4, wherein the infrared (IR) radiation is short-wavelength infrared or mid-wavelength infrared radiation.

6. Method according to any one of claims 1 to 5, wherein the radiant power of the infrared radiation is from 15 to 70 kW.

7. Method according to any one of claims 1 to 6, wherein the step (iv) is carried out at an ambient temperature from 0 to 50°C.

8. Laminate obtainable by the method according to any one of claims 1 to 7.

9. Laminate according to claim 8, wherein the crosslinked adhesive medical silicone is in form of a gel.

10. Medical product comprising the laminate according to claim 8 or 9.

11. Medical product according to claim 10 being a wound dressing.

12. Medical product according to claim 10 or 11, wherein the layer of the crosslinked silicone is the wound contact layer.

13. Use of an infrared radiation source for crosslinking siloxane prepolymers, preferably alkenyl-containing poly(organo)siloxane(s) and Si-H-containing poly(organo)siloxane(s), to obtain an adhesive crosslinked polymer.

14. Use of claim 13 wherein the adhesive crosslinked polymer is comprised by medical product.

15. Apparatus comprising an infrared radiation source configured to carry out the method according to any one of claims 1 to 7.

Figure 1:

Crosslinking indicator tan delta versus time [days]

—○—IR 25%    --□--IR 35%    ---▲---Oven 140-30

Figure 2

peel strength versus time [days]

—○—IR 25%    --□--IR 35%    ---▲---Oven

Figure 3

**PE release force VS time [days]**

—◇—IR 25%    —◻—IR 35%    —△—Oven

Figure 4

**Release force at high speed - tests 2 weeks after coating**

—◇—IR 25%    —◻—IR 35%    —△—Oven 140°C -30

Figure 5

Removal of perforated PE- Spillker process

Figure 6

PE Release Force (180° -300 mm/min)

Figure 7

Figure 8

Figure 9

| | Europäisches Patentamt European Patent Office Office européen des brevets | **EUROPEAN SEARCH REPORT** | **Application Number** EP 24 31 5367 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/297544 A1 (MOINE CAROLINE [FR] ET AL) 24 September 2020 (2020-09-24) <br> * paragraph [0035] - paragraph [0074] * <br> * paragraph [0127] * <br> * paragraph [0194] * <br> * paragraph [0196] - paragraph [0198] * <br> ----- | 1-15 | INV. A61L15/58 |
| X | US 2019/099517 A1 (MOINE CAROLINE [FR] ET AL) 4 April 2019 (2019-04-04) <br> * paragraph [0036] - paragraph [0071] * <br> * paragraph [0118] * <br> * paragraph [0189] * <br> ----- | 1-15 | |
| X | EP 1 637 564 A1 (WACKER CHEMIE AG [DE]) 22 March 2006 (2006-03-22) <br> * paragraph [0008] * <br> * paragraph [0040] * <br> * paragraph [0044] * <br> * paragraph [0029] * <br> ----- | 1-15 | |
| | | | **TECHNICAL FIELDS SEARCHED (IPC)** A61L |

The present search report has been drawn up for all claims

| Place of search | Date of completion of the search | Examiner |
|---|---|---|
| The Hague | 14 January 2025 | Fort, Marianne |

EPO FORM 1503 03.82 (P04C01)

## EP 4 686 485 A1

## ANNEX TO THE EUROPEAN SEARCH REPORT
## ON EUROPEAN PATENT APPLICATION NO.

EP 24 31 5367

14-01-2025

| Patent document cited in search report | | Publication date | Patent family member(s) | | Publication date |
|---|---|---|---|---|---|
| US 2020297544 | A1 | 24-09-2020 | CN | 109476916 A | 15-03-2019 |
| | | | DK | 3430086 T3 | 06-04-2020 |
| | | | EP | 3430086 A1 | 23-01-2019 |
| | | | FR | 3048885 A1 | 22-09-2017 |
| | | | JP | 6679749 B2 | 15-04-2020 |
| | | | JP | 2019516811 A | 20-06-2019 |
| | | | KR | 20180125529 A | 23-11-2018 |
| | | | US | 2020297544 A1 | 24-09-2020 |
| | | | WO | 2017158249 A1 | 21-09-2017 |
| US 2019099517 | A1 | 04-04-2019 | CN | 109415563 A | 01-03-2019 |
| | | | DK | 3430087 T3 | 06-04-2020 |
| | | | EP | 3430087 A1 | 23-01-2019 |
| | | | FR | 3048886 A1 | 22-09-2017 |
| | | | JP | 6859359 B2 | 14-04-2021 |
| | | | JP | 2019511607 A | 25-04-2019 |
| | | | KR | 20180125163 A | 22-11-2018 |
| | | | US | 2019099517 A1 | 04-04-2019 |
| | | | WO | 2017158250 A1 | 21-09-2017 |
| EP 1637564 | A1 | 22-03-2006 | CN | 1749323 A | 22-03-2006 |
| | | | DE | 102004044943 A1 | 23-03-2006 |
| | | | EP | 1637564 A1 | 22-03-2006 |
| | | | JP | 2006083392 A | 30-03-2006 |

EPO FORM P0459